# EUROPÄISCHE PATENTANMELDUNG

(11) **EP 2 312 294 A2**
(43) Veröffentlichungstag der Anmeldung: **20.04.2011**
(21) Anmeldenummer: 10177556.7
(22) Anmeldetag: 20.09.2010
(51) Int. Cl.: G01N 11/00

(54) **Verfahren zur Prüfung der Eigenschaften eines viskoelastischen Materials durch Kombination von rheologischer mit dielektrischer Prüfmethode**

(30) Priorität: 14.10.2009 DE 102009044247
(71) Anmelder: ContiTech AG, 30165 Hannover (DE)
(72) Erfinder: Sostmann, Stefan, 30855 Langenhagen (DE); Herrmann, Wolfram, 31515 Wunstorf (DE)
(74) Vertreter: Finger, Karsten

(57) **Zusammenfassung**

Die Erfindung betrifft ein Verfahren zur Prüfung der Eigenschaften eines viskoelastischen Materials, wobei erfindungsgemäß eine rheologische Prüfmethode zur Erfassung der dynamisch-mechanischen Eigenschaften mit einer dielektrischen Prüfmethode zur Erfassung der dielektrischen Eigenschaften eines viskoelastischen Materials kombiniert wird.

Nach einer bevorzugten Verfahrensvariante wird im Rahmen der rhelogischen Prüfmethode die Messkammer eines Torsionsrheometers mit Elektroden ausgestattet, die es erlauben, während der dynamisch aufgebrachten Lasten die dielektrischen Eigenschaften zu verfolgen. Dabei wird insbesondere ein bikonischcs Zwciplattcnsystcm eingesetzt.

Das Verfahren kommt insbesondere bei unvulkanisierten Kautschukmischungen zur Anwendung.

## Beschreibung

Die Erfindung betrifft ein Verfahren zur Prüfung der Eigenschaften eines viskoelastischen Materials. Die Viskoelastizität ist die Sammelbezeichnung für das viskoelastische Verhalten von Festkörpern, die häufig unter Druckbelastung das Phänomen des Kriechens zeigen. Von besonderer Bedeutung sind dabei Viskopolymere, insbesondere wiederum Viskoelastomere. Diesbezüglich wird beispielsweise auf folgende Literatur verwiesen: Ferry, Viscoelastic Properties of Polymers, S. 33, New York; John Wiley & Sons 1980.

Dynamisch-mechanische Rheometer-Tests sind standardmäßig in der Prüfung viskoelastischer Materialien im Einsatz. Es gibt dabei eine Vielzahl verschiedener Ausführungen dieser Messtechnik, unterschiedlich bezüglich der Art der Belastung (Druck, Zug, Scherung, Torsion), der Form der Probenhalter und Probenkammer, der aufgebrachten Belastungen und weiterer Bedingungen.

Unter Rheometern werden Rotationsviskometer verstanden, die über erweiterte Steuerungsmöglichkeiten verfügen. Neben der üblichen Steuerung der Drehzahl können die Messungen drehmomentabhängig, winkelabhängig und mit oszillierender Rotation gesteuert werden. Sie erlauben dadurch die sehr genaue Untersuchung spezieller Eigenschaften, beispielsweise der Fließgrenze. Als besonders geeignet haben sich Torsionsrheometer erwiesen, bei denen dynamische Lasten auf eine zwischen zwei Rotoren befindlichen Polymerschicht, insbesondere Elastomerschicht, aufgebracht werden und so dynamisch-mechanische Kennwerte des viskoelastischen Materials gewonnen werden.

Zur dielektrischen Charakterisierung von viskoelastischen Materialien gibt es ebenfalls gängige Messtechniken auf dem Markt. Hier gibt es die verschiedensten beschriebenen Ausführungen für Probenhalter und Messaufbauten, die aber allesamt das Material "nur" unter statischen Bedingungen beurteilen.

Im Rahmen einer Weiterentwicklung besteht die Aufgabe der Erfindung darin, ein Verfahren bereitzustellen, das die Erfassung eines umfassenden Eigenschaftsbildes eines viskoelastischen Materials ermöglicht, um eine genaue Vorhersage der Produkteigenschaften von daraus gefertigten Artikeln zu ermöglichen.

Gelöst wird diese Aufgabe dadurch, dass eine rheologische Prüfmethode zur Erfassung der dynamisch-mechanischen Eigenschaften mit einer dielektrischen Prüfmethode zur Erfassung der dielektrischen Eigenschaften des viskoelastischen Materials kombiniert wird.

Das Prüfen der dynamisch-mechanischen Eigenschaften an viskoelastischen Materialien erlaubt die Vorhersage der Produkteigenschaften von daraus gefertigten Artikeln. Sie ist für die Materialentwicklung ganz entscheidend und zeigt zudem neben den Rezepturbezogenen Kennwerten auch ganz empfindlich die ,,Verarbeitungshistorie" des Materials an. So kann man die Prüfung zur Prozess- und Rezepturkontrolle verwenden. Aus den Messungen erkennt man empfindlich Änderungen in der Zusammensetzung der Inhaltsstoffe, die Qualität der Verteilung der Inhaltsstoffe ineinander und den Zustand (Abbaugrad, Schädigungen usw.) des Elastomers.

Durch die erfindungsgemäße Kombination dieses Testkonzeptes mit dielektrischen Messungen kann man noch aufgelöster die Eigenschaften des Materials während der dynamischen Belastung verfolgen. Die dielektrischen Messungen fokussieren dabei stark auf die Verteilung und die Wechselwirkung der Füllstoffpartikel untereinander und können so die dynamisch-mechanischen Messdaten in ihrer Aussage noch deutlich verbessern. Besonders vorteilhaft ist es, wenn man die Messkammer eines Torsionsrheometers, insbesondere wiederum in Form eines bikonischen Zweiplattensystems (z.B. im PPA der Firma Alpha Technologies), mit Elektroden ausstattet, die es erlauben, während der dynamisch aufgebrachten Lasten die dielektrischen Eigenschaften zu verfolgen. Dabei gibt es verschiedene Möglichkeiten der Elektrodenart und Elektrodenkontaktierung. Auch das RPA als Prüfgerät, das bei gleicher Funktion ein anderer Bautyp ist, wäre ergänzend zu nennen.

Das erfindungsgemäße Verfahren wird insbesondere zur Prüfung der Eigenschaften einer unvulkanisierten Kautschukmischung, enthaltend wenigstens eine Kautschukkomponente und Mischungsingredienzien, eingesetzt. Als Kautschukkomponenten sind insbesondere zu nennen:
Ethylen-Propylen-Kautschuk (EPM)
Ethylen-Propylen-Dien-Kautschuk (EPDM)
Nitrilkautschuk (NBR)
(teil)hydrierter Nitrilkautschuk (HNBR)
Fluor-Kautschuk (FKM)
Chloropren-Kautschuk (CR)
Naturkautschuk (NR)
Styrol-Butadien-Kautschuk (SBR)
Isopren-Kautschuk (IR)
Butylkautschuk (IIR)
Brombutylkautschuk (BIIR)
Chlorbutylkautschuk (CIIR)
Butadien-Kautschuk (BR)
Chloriertes Polyethylen (CM)
Chlorsulfoniertes Polyethylen (CSM)
Polyepichlorhydrin (ECO)
Ethylen-Vinylacetat-Kautschuk (EVA)
Acrylat-Kautschuk (ACM)
Ethylen-Acrylat-Kautschuk (AEM)
Silikonkautschuk (MQ, VMQ, PVMQ, FVMQ; DE 10 2006 058 470 A1)
Fluorierter Methylsilikonkautschuk (MFQ)
Perfluorinierter Propylen-Kautschuk (FFPM)
Perfluorcarbon-Kautschuk (FFKM)
Polyurethan (PU)

Die vorgenannten Kautschuktypen können unverschnitten sein. Auch der Einsatz eines Verschnittes, insbesondere in Verbindung mit einem der vorgenannten Kautschuktypen, beispielsweise ein NRBR-Verschnitt oder ein BR/SBR-Verschnitt, ist möglich.

Von besonderer Bedeutung sind: EPM, EPDM, SBR, BR, CR, NR, NBR, HNBR, FKM, ACM oder AEM.

Der Schwerpunkt liegt auf der Untersuchung der unvulkanisierten Kautschukmischung einer zu einem späteren Zeitpunkt noch vulkanisierbaren Rezeptur und damit der Vorhersage von Produkteigenschaften im vulkanisierten Zustand.

Die üblichen Mischungsingredienzien umfassen wenigstens einen Vernetzer oder ein Vernetzersystem (Vernetzungsmittel und Beschleuniger). Weitere Mischungsingredienzien sind zumeist noch ein Füllstoff und/oder ein Verarbeitungshilfsmittel und/oder ein Weichmacher und/oder ein Alterungsschutzmittel sowie gegebenenfalls weitere Zusatzstoffe (z.B. Farbpigmente, Fasern). Diesbezüglich wird auf den allgemeinen Stand der Kautschukmischungstechnologie verwiesen.

Auch thermoplastische Elastomere (TPE) können hiermit untersucht werden. Hinsichtlich einer bevorzugten TPE-Mischung wird insbesondere auf die Offenlegungsschrift DE 100 04 632 A1 verwiesen.

Die aus diesen viskoelastischen Materialien hergestellten Artikel sind insbesondere:
- Schläuche (z.B. Kraftfahrzeugschlauch, Schwimmschlauch);
- Luftfederbälge (Axialbalg, Kreuzlagenbalg);
- Kompensatoren;
- Antriebsriemen (Flachriemen, Keilriemen, Keilrippenriemen, Zahnriemen);
- Fördergurte (Textilgurt, Stahlseilgurt);
- Reifen;
- Gummierte Stoffe (z.B. Behälterstoff, Bootsstoff, Schutzstoff),
- Innenraumverkleidungen;
- Buchsen und Lager;
- Dichtungen in Form eines Extrudates oder eines Formartikels.

## Patentansprüche

1. Verfahren zur Prüfung der Eigenschaften eines viskoelastischen Materials, **dadurch gekennzeichnet, dass** eine rheologische Prüfmethode zur Erfassung der dynamisch-mechanischen Eigenschaften mit einer dielektrischen Prüfmethode zur Erfassung der dielektrischen Eigenschaften des viskoelastischen Materials kombiniert wird.

2. Verfahren nach Anspruch 1, **dadurch gekennzeichnet, dass** im Rahmen der rheologischen Prüfmethode die Messkammer eines Torsionsrheometers mit Elektroden ausgestattet wird, die es erlauben, während der dynamisch aufgebrachten Lasten die dielektrischen Eigenschaften zu verfolgen.

3. Verfahren nach Anspruch 2, **dadurch gekennzeichnet, dass** ein bikonisches Zweiplattensystem eingesetzt wird.

4. Verwendung eines Verfahrens nach einem der Ansprüche 1 bis 3 zur Prüfung der Eigenschaften einer Kautschukmischung, enthaltend wenigstens eine Kautschukkomponente und Mischungsingredienzien.

5. Verwendung eines Verfahrens nach Anspruch 4, wobei die Kautschukkomponente Ethylen-Propylen-Kautschuk (EPM), Ethylen-Propylen-Dien-Kautschuk (EPDM), Nitrilkautschuk (NBR), (teil)hydrierter Nitrilkautschuk (HNBR), Fluor-Kautschuk (FKM), Chloropren-Kautschuk (CR), Naturkautschuk (NR), Styrol-Butadien-Kautschuk (SBR), Isopren-Kautschuk (IR), Butylkautschuk (IIR), Brombutylkautschuk (BIIR), Chlorbutylkautschuk (CIIR), Butadien-Kautschuk (BR), Chloriertes Polyethylen (CM), Chlorsulfoniertes Polyethylen (CSM), Polyepichlorhydrin (ECO), Ethylen-Vinylacetat-Kautschuk (EVA), Acrylat-Kautschuk (ACM), Ethylen-Acrylat-Kautschuk (AEM), Silikonkautschuk (MQ, VMQ, PVMQ, FVMQ), Fluorierter Methylsilikonkautschuk (MFQ), Perfluorinierter Propylen-Kautschuk (FFPM), Perfluorcarbon-Kautschuk (FFKM) oder Polyurethan (PU) ist, wobei die vorgenannten Kautschuktypen verschnittfrei oder in einem Verschnitt, insbesondere in Verbindung mit einem der vorgenannten Kautschuktypen, zum Einsatz gelangen.

6. Verwendung eines Verfahrens nach Anspruch 4 oder 5 zur Prüfung der Eigenschaften einer unvulkanisierten Kautschukmischung.
